# EUROPEAN PATENT APPLICATION

(11) **EP 3 756 673 A1**
(43) Date of publication of application: **30.12.2020**
(21) Application number: 19744544.8
(22) Date of filing: 07.01.2019
(51) Int. Cl.: A61K 31/7105, C12N 15/113, A61K 9/127, A61P 35/00

(54) **NANOMETRIC PHARMACEUTICAL COMPOSITION FOR RELEASE OF INTERFERING RNA MOLECULES AND USE THEREOF**

(30) Priority: 24.01.2018 BR 102018001541
(71) Applicant: Universidade Federal Do Rio Grande Do Sul, 90040060 Porto Alegre-RS (BR); Universidade Federal de Ciências da Saúde de Porto Alegre - UFCSPA, Porto Alegre RS (BR); Universidade Federal de Pelotas, Pelotas RS (BR); Universidade Federal Do Pampa - Unipampa, 900 Bairro Centro Bagé RS (BR)
(72) Inventor: BRAGANHOL, Elizandra, 90520-000 Porto Alegre (BR); OLIVEIRA BATTASTINI, Ana Maria, Porto Alegre (BR); TEIXEIRA, Helder, Porto Alegre (BR); STEFANI, Marco Antônio, Porto Alegre (BR); BRUXEL, Fernanda, Uruguaiana (BR); MARIA SPANEVELLO, Roselia, Pelotas (BR); CARDOSO TEIXEIRA, Fernanda, Pelotas (BR); HOFSTATTER AZAMBUJA, Juliana, Porto Alegre (BR)
(74) Representative: Petraz, Gilberto Luigi
(86) International application number: PCT/BR2019/050004
(87) International publication number: WO 2019/144209

(57) **Abstract**

The present invention relates to a nanometric-scale composition in the form of liposomes or nanoemulsions containing strands of interfering RNA (siRNA) capable of silencing the gene responsible for the expression of superexposed adhesion proteins in tumours, such as the enzyme ecto-5'-nucleotidase/CD73 (CD73) which is superexpressed in multiform glioblastoma and in other types of tumour. The binding of the liposomes or nanoemulsions to the siRNA promotes a site-directed release complex capable of being incorporated into various types of formulation, such as an intratumoral or intravenous injection or nasal administration.

## Description

### Field of the invention

The present invention is located among the nanotechnology, Pharmacy and genetics fields, being related to a composition in nanometric scale aiming the delivery of molecules of RNA of interference able to silencing genes responsible for the expression of proteins and enzymes overexposed in tumors. The composition of formulation contains lipossomes or nanoemulsions which interact efficiently with RNA of interference, forming complexes of target release. The composition allows compatibility with a variety of pharmaceutical formulations, in addition to be safer than the traditional methods.

### History of invention

Cancer is a group of diseases that involve the anormal cell growth, with potential to invade and spread to other organs of the body beyond the original/primary site. A cellular mass exhibiting anormal groth is named tumor.

Glioma is a general word to identify a group of brain tumors that exhibit phenotipic and genetic characteristics similar to glial cells. A variety of tumors could be considered gliomas, as the glioblastoma multiforme. The majority of brain tumors that exhibit high rate of growth are grade IV gliomas.

Glioblastoma multiforme, also known as glioblastoma, is the most common and aggressive type of malignant brain tumor that affects humans. The signs and initial symptoms are nonspecific and can include headache, personality changes, nausea and stroke-like symptoms. The worsening of symptoms is generally rapid, and may evolve into unconsciousness. The prognosis for patients with this type of tumor is poor and the mean survival after diagnosis is 12 to 15 months.

Current treatment methods basically consist of neurosurgery, the use of substances that inhibit tumor growth, or the inhibition of angiogenesis which is necessary for the demand for nutrients of this type of tumor, that is, in the cytotoxic action that prevents the replication - and, consequently, growth - of the tumor.

Neurosurgery to remove tumor cells, despite being an effective method of treatment, has some common complications, such as the location of the tumor and the degree of invasiveness. Depending on the region of growth, some tumors may be inoperable, or even operable, but with high surgical danger and/or significative post-surgical discomfort for the patient.

The use of substances that inhibit tumor growth also has disadvantages. The first-line drug for the treatment of glioblastoma multiforme is temozolomide, an oral chemotherapy and anti-neoplastic alkylating agent. Despite being a new drug and with few side effects, it still exhibits risks, since it is genotoxic and teratogenic. Its effectiveness is also limited due to the high speed of tumor replication, and its main use is to increase patient survival.

In relation to formulations containing the growth-inhibiting substances already mentioned, the majority of them are manipulated as oral or intravenous formulations. Depending on the substance to be delivered, some disadvantages can also be noticed. In the case of temozolomide, for example, the oral administration should be high enough for overcome the primary hepatic metabolism, in addition to spreading into the bloodstream, inhibiting the cell division of other healthy cells and generating the discomfort of chemotherapy drugs, a disadvantage also present in intravenous administration. Other drugs may also have the problem of crossing the blood-brain barrier, since gliobastoma is found in the brain.

Ideally, an antineoplastic formulation should be able to deliver only the effective amount of the chemotherapeutic at the specific site of action, in addition to being able to cross the blood-brain barrier and easy to be administer.

In addition, the toxicity of the chosen chemotherapeutic active must be rigorously analyzed. Ideally, the chemotherapeutic active should be effective only against tumor cells, selectively inhibiting its cell replication.

However, current antineoplastic has systemic action and carry high dosages of toxic chemotherapeutic, resulting significant side effects for patients.

The present invention aims to address all the problems explained above. The invention comprises a system of liposomes or nanoemulsion, containing a specific and effective amount of chemotherapeutic active with genetic action. The invention uses interference RNA sequences capable of silencing the gene responsible for the transcription of the adhesion protein and adenosine-generating enzyme, a tumor-promoting molecule, ecto-5'-nucleotidase/CD73, overexpressed in several tumors, including glioblastoma.

Thus, the present invention addresses not only a formulation capable to carry the effective amount of the drug as well as a drug specfic against tumor characteristics.

### Background of the invention:

The document WO2004/079013 describes a method of diagnosing and predicting the stage of pancreatic cancer which comprises the detection of ecto-5'-nucleotidase expression and activity in a sample of cancer cells and further comparing the results with normal cells. The document also reports compositions for the treatment of tumors containing fragments of interfering RNA, and that these compositions can be liposomes and emulsions. However, there is no mention of the treatment of glioblastomas or the nanometric size of the formulations.

The document PL 0709506-6 describes the use of interfering RNA for inhibiting the expression of spleen tyrosine kinase mRNA (SYK), particularly for the treatment of patients who have a SYK related inflammatory condition or running risk of developing an inflammatory condition related to it, such as allergic conjunctivitis, eye inflammation, dermatitis, rhinitis, asthma, allergy, or mast cell disease.

The document PL 0619738-8 describes isolated interference RNA (siRNA) sequences, comprising a strand of sense RNA and a strand of complementary antisense RNA, which together form an RNA duplex, with fragments of 14 to 30 contiguous nucleotides of the nucleotide sequence of exon F of the gene encoding the protein myosin V. The document also describes compositions comprising at least one siRNA and the use of at least that siRNA as a cosmetic or therapeutic agent for skin depigmentation.

The paper "Anti-CD73 antibody therapy inhibits breast tumor growth and metastasis", published in the Proceedings of the National Academy of Sciences of the United States of America (PNAS) on January 26, 2010, describes, as it supposes the title, the development of a selective monoclonal antibody for the ecto-5'-nucleotidase adhesion protein, which is overexpressed in breast tumors.

Thus, none of the above describes, at the same time, a liposome/nanoemulsion system, containing specific interfering RNA fragments, and applied to inhibit the activity of the adhesion protein and extracellular adenosine-generating enzyme, the ecto- 5'-nucleotidase in glioblastomas.

Considering the information/limitations described above, the inventors developed the present invention, which provides solutions to various technical problems already presented. This document describes antineoplastic nanometric formulations, which aim to overcome several problems mentioned above and promote safety and efficacy in the treatment of glioblastomas.

### Summary of the invention

The present invention refers to a nanometric scale formulation containing interference RNA strands capable of silencing genes that regulate the expression of adhesion proteins overexposed in some types of tumors.

In a first aspect, the present invention provides a nanometric composition consisting of liposomes or nanoemulsions containing strands of interfering RNA.

In a second aspect, the present invention provides the use of the said composition to silence genes that regulate the expression of overexposed proteins in tumors.

In a third aspect, the present invention presents liposomes or nanoemulsion complexed to interfering RNA capable of crossing the blood-brain barrier and being site specific.

In a fourth aspect, the present invention provides a pharmaceutically acceptable formulation containing the nanometric composition.

In one concretization, the pharmaceutically acceptable formulation is an intratumoral injection, an intravenous injection or a nasal spray.

These and other aspects, including the characteristics and advantages of the invention will become even more evident to specialized reviewers from the detailed description below and the attached claims.

### Brief Description of the Figures

Figure 1 shows the analysis of ecto-5'-NT/CD73 activity and expression in C6 glioma cells and the effect of AMPCP on cell proliferation.
Figure 2 shows the evaluation of ecto-5'-NT/CD73 silencing by siRNA-CD73 specific sequences.
Figure 3 shows the morphological characterization of the nanoemulsion (NE)/siRNA-CD73 complexes.
Figure 4 shows the optimization of C6 glioma transfection using NE/siRNA complexes.
Figure 5 shows the cytotoxicity analysis of the NE/siRNA-GFP complex in astrocyte cultures.
Figure 6 shows the evaluation of the silencing of the enzyme ecto-5'NT/CD73 by NE/siRNA-CD73 complexes.
Figure 7 shows the analysis of NE/siRNA-CD73 treatment on C6 glioma cell viability.

### Detailed Description of the Invention

The present description aims to deepen the details about the inventive concept, provide examples that facilitate the cognition/understanding of it and provide precise technical data on some of the ways to concretize the inventive concept of the invention. The detailed description also aims to avoid the repetition, by third parties, of the extensive experimentation, financial investments, time and intellectual activity that the inventors/depositor made to solve the technical problems now solved.

To avoid questions as to the interpretation, any feature described in one aspect of the present invention can be used in another aspect of the invention. The word "comprising" is intended to mean "including", but not necessarily "consisting of" or "composed only of." In other words, the steps or options listed need not be exhaustive. Note that the examples provided in the description below are intended to clarify the invention and should not per se be interpreted as limiting the scope of the invention.

In a first aspect, the present invention provides a nanometric pharmaceutical composition comprising liposomes or nanoemulsion containing interference RNA strands.

In one concretization of the nanometric pharmaceutical composition, the interfering RNA strands are composed of two identical sequences of 19 nucleotides in reverse orientation, separated by a space of 9 base pairs, defined according to SEQ ID No: 1 , SEQ ID No: 2 and SEQ ID No: 3.

In one concretization of the nanometric pharmaceutical composition, it further comprises a pharmaceutically acceptable excipient.

In one concretization of the nanometric pharmaceutical composition, it is in the form of an intratumoral, parenteral injection or nasal spray.

In a second aspect, the present invention provides the use of said nanometric pharmaceutical composition to prepare a drug to silence genes responsible for the expression of overexposed proteins in tumors.

In one concretization of the use, the silenced gene is 5'-nucleotidase-ecto.

In one concretization of the use, the protein to be regulated is ecto-5 'nucleotidase/CD73.

In one concretization of the use, the tumor is the glioblastoma multiforme.

In one concretization of the use, the composition is administered in the form of an intratumoral, parenteral injection or nasal spray.

The treatment of glioblastoma multiforme remains a challenge for oncology. The therapies currently offered are only palliative and the average survival of diagnosed patients is only 12 months.

Therefore, the proposed invention aims to offer a new therapeutic strategy for the treatment of glioblastoma multiforme that can also be used for other neoplasms that have not yet been cured or refractory to the available treatments.

The therapy target is the enzyme and adhesion protein ecto-5 'nucleotidase/CD73 (CD73). Increased expression and enzyme activity of this protein has been widely reported in tumor tissues, including glioblastomas and is associated with the increase of the tumor malignancy characteristics, such as cell migration, adhesion, invasion, angiogenesis and tumor escape from the immune system. Thus, strategies to decrease the expression and/or activity of CD73 could be useful for the treatment of glioblastoma multiforme and also of other neoplasms that present an increased expression of this target.

An innovative way of silencing gene expression is the use of interference RNA (siRNA) sequences for a given target. Interference RNA is a mechanism exerted from a double RNA strand of approximately 19-23 nucleotides that triggers the cleavage of specific messenger RNA sequences, resulting in the inhibition of gene expression in the translation phase or hindering the transcription of specific genes. The end result is a decrease in the expression of the target protein, which, in this proposal, is CD73.

Although siRNA technology is very promising, its use in the clinic is limited by some factors, including the difficulty to access the central nervous system imposed by the blood-brain barrier (BBB) and the degradation of siRNA sequences by endogenous nucleases.

In order to overcome the aforementioned problems, siRNA sequences can be administered locally, via intracerebral/intratumoral injections, intranasally or systemically, intravenously, using liposomal systems or nanoemulsions as delivering carriers. Said formulations interact efficiently with siRNA, forming complexes that potentially cross cell and blood-brain barriers and facilitate targeted site release, cell uptake and interaction with the intracellular target of siRNA sequences.

In addition, the use of non-viral vectors such as liposomes/nanoemulsions has been considered a more attractive alternative when compared to viral vectors, due to aspects of biosafety.

Briefly, the present invention proposes a nanometric scale composition, containing liposomes or a nanoemulsion complexed to specific interference RNA (siRNA) strands, capable of silencing the 5'-nucleotidase-ecto gene, also known as NT5E or ID gene: 4907. This gene is responsible for the expression of the ecto-5'nucleotidase/CD73 (CD73), an adhesion protein overexpressed in glioblastoma multiform and also in other types of tumors.

### Primary and Cell Line Cultures:

The glioma cell line (C6) was obtained from the ATCC (American Type Cell Collection, USA) and was grown in Dulbecco's Modified Eagle's Medium (DMEM), a sterile culture medium containing glucose, L-glutamine and bicarbonate of sodium, and 5% fetal bovine serum (FBS) in a cell incubator at 37°C and 5% CO₂/95% humidity, according to the standard protocol for cell culture maintenance.

Primary cultures of astrocytes were prepared from rat brain tissue. The cortex of newborn Wistar rats (1-2 days old) were removed and mechanically dissociated in a balanced saline solution free of Ca⁺² and Mg⁺² (pH 7.4), containing 137 mM NaCl, 5.36 mM KCI, 0.27 mM Na₂HPO₄, 1.1 mM KH₂PO₄, and 6.1 mM glucose. After centrifugation at 1,000 g for 5 min, the pellet was suspended in culture medium (pH 7.6) containing 1% DMEM, 8.39 mM HEPES buffer (4- (2-hydroxyethyl) -1 - piperazine ethanesulfonic acid) pH 7.6, 23.8 mM NaHCO₃, 0.1% fungizone® (Amphotericin B, antifungal), 0.032% antibiotic garamycin and 10% FBS. The cells were seeded at a density of 1.5 x 10⁵ cells/cm² in 24-well plates pre-treated with poly-L-lysine. Cultures were also maintained at 5% CO₂ / 95% air at 37°C.

Further, the cells were treated with 5'-adenosine-(α,β-methylene)-diphosphate (AMPCP, Sigma, USA), a selective inhibitor of CD73 and used to study the regulation of adenosinergic signaling through this protein. AMPCP was dissolved in water at a concentration of 100 mM (stock solution) and subsequently diluted in DMEM 5%/FBS to obtain the final concentrations (1, 10 and 100 µM). C6 glioma cells (2x10⁴ cells/well) were seeded in 24-well plates. After 24 h, cultures were treated with AMPCP for 48 h. Controls were exposed only to the culture medium (DMEM/5% FBS).

After the treatment with AMPCP, the medium was removed, the cells were washed with PBS buffer solution (phosphate buffered saline, solution containing NaCl and NaHPO₄), trypsinized with 200 µl of 0.25% trypsin solution and counted in neubauer chamber.

Following the treatment, the cells were submitted to enzyme activity, cell viability, and immunocytochemistry for CD73 staining experiments, in order to determine the reliability of the tests performed with the NE/siRNA complexes.

The enzymatic activity of C6 cells was determined in an incubation medium (2 mM MgCl₂, 120 mM NaCl, 5 mM KCl, 10 mM glucose, 20 mM HEPES [pH 7.4], and 2 mM AMP [adenosine monophosphate]) at 37°C for 10 min. The inorganic phosphate (Pi) released by the enzyme activity of CD73 was measured, and the protein concentration was evaluated by Malachite Green and Coomassie Blue methods, respectively. The enzyme activity was expressed as µmol Pi released/min/mg of protein.

To perform the cell viability assay, C6 glioma cells or primary astrocytes (5x10³ cells/well) were exposed to the NE/siRNA complexes and after 48 h the cell viability was determined by the MTT assay, a colorimetric assay where a yellow tetrazolic compound is reduced to a purple formazan by living/viable cells.

To perform the immunocytochemistry for CD73, C6 glioma cultures were fixed in acetone/formalin solution and washed 3 times for 10 min each with PBS. The cells were incubated with the blocking solution (7% FBS prepared in PBS containing 0.2% Tween-20 [polysorbate, non-ionic surfactant]) for 45 min at room temperature. Afterwards, the cells were incubated for 90 min with the primary polyclonal rabbit anti-rat antibody CD73 (rNu-9L15, 1: 1,000) diluted in 7% FBS prepared in PBS containing 0.2% Tween-20. The cells were then incubated with the FITC-conjugated secondary anti-rabbit antibody (affinity-purified antibodies with a well-characterized specificity, providing greater sensitivity through signal amplification, since multiple secondary antibodies can bind to a single antibody primer; fluorescein isothyanate conjugate; 1:1,000) for 60 min at room temperature. The images were captured using a digital camera attached to a microscope.

### Development of siRNA sequences for the adhesion protein ecto-5 'NT/CD73:

The DNA sequences encoding CD73 (gene ID: 4907) were selected to perform the design of siCD73 sequences. All designed sequences were evaluated by BLAST (NCBI) in order to confirm specific homology with the target gene.

Sense and antisense synthetic oligonucleotides constituted the template for the generation of an RNA molecule composed of two identical sequences of 19 nucleotides in inverted orientation, separated by a space of 9 base pairs, forming a siRNA harpin, according to Table 1 and the sequences defined as SEQ ID No: 1, SEQ ID No: 2 and SEQ ID No: 3, respectively. As siRNA controls, scramble sequences absent in the genome database of humans, rats or mice were used.

**TABLE 1**

| **siRNA** | **Sequences** | |
|---|---|---|
| **GFP** | *Sense* | 5'[Phos]rCrArGrGrCrUrArCrUrUrGrGrArGrUrGrUrArUdTdT3' |
| | *Antisense* | 5'[Phos]rArUrArCrArCrUrCrCrArArGrUrArGrCrCrUrGdTdT3' |
| **961** | *Sense* | 5'[Phos]rGrCrCrArUrCrArArArGrCrArGrArCrArUrUrArArC3' |
| | *Antisense* | 5'[Phos]rUrArArUrGrUrCrUrGrCrUrUrUrGrArUrGrGrCrUrG3' |
| **980** | *Sense* | 5'[Phos]rArCrCrArGrUrGrGrArGrGrArUrArArArArUrUrArG3' |
| | *Antisense* | 5'[Phos]rArArUrUrUrUrArUrCrCrUrCrCrArCrUrGrGrUrUrA3' |

### Preparation and characterization of nanoemulsions (NE):

NE composed of 8% (w/w) medium chain triglycerides, 2% (w/w) egg lecithin, 0.132% (w/w) DOTAP (N- [1- (2.3 -dioleoyloxy) propyl] -N, N, N trimethylammonium methyl sulfate, a liposomal transfection agent), 2.25% (w / w) glycerol and water to complete 100% (w/w) were prepared by spontaneous emulsification. Briefly, an ethanolic solution containing the components of the oil phase was slowly added to the aqueous phase containing glycerol under moderate stirring. The excess solvent mixture (ethanol/water) was removed under reduced pressure at 50°C until reaching the desired final volume (5 mL). The final concentration of cationic lipid was 2 mM, as previously optimized.

### Preparation and characterization of NE / siRNA complexes:

The adsorption of the siRNA-GFP, siRNA-CD73-961 or siRNA-CD73-980 sequences on cationic NE was performed at the end of NEs production, resulting the formation of the NE/siRNA-GFP, NE/siRNA-CD73-961 or NE/siRNA-CD73-980 complexes. Increasing concentrations of NE were added to aqueous solutions of siRNA sequences (1 µM-final concentration) and incubated for 15 min at room temperature.

NEs were chosen as non-viral vectors for the delivery of siRNA-CD73 sequences to specific targets. After the adsorption of siRNA-CD73 sequences in cationic NE, the physicochemical properties of the complexes were determined (Table 2).

Complexes were prepared in three different positive/negative charge ratios (+0.1/-; +0.5/-; +2/-). The charge ratios were calculated considering the number of positive charges of the cationic lipid present in the NE and the number of negative charges of the phosphate groups of the siRNA sequences.

The average of particle size, zeta potential and polydispersity index (PDI) were determined by photon correlation spectroscopy and electrophoretic mobility (Zetasizer Nano ZS, Malvern Instrument, UK), at 20°C. The complex was adequately diluted in water for size and PDI determinations or in 1 mM NaCl solution for zeta potential measurements.

The morphology of the NE/siRNA complexes were also evaluated as described above. The average of particle size of NE range from 249.9 to 526.52 nm and the PDI range from 0.1 to 0.6. The zeta potential values of the NE/siRNA complexes were lower than zero, resulting in variations in particle size among the preparations. In general, the characteristics exhibited by the formulations are in agreement with other NE systems reported in the literature.

**TABLE 2**

| **siRNA** | **Load Ratio** | **Average Diameter (nm)** | **Zeta Potential (mV)** | **Polydispersity Index** |
|---|---|---|---|---|
| **NE** | - | 188.48 ± 25.60 | 45.5 ± 5.13 | 0.1412 ± 0.059 |
| **NE/siRNA-GFP** | +0.1/- | 348 ± 12.26 | -42.1 ± 4.12 | 0.302 ± 0.074 |
| | +0.5/- | 323.5 ± 105 | -32.4 ± 10.14 | 0.427 ± 0.154 |
| | +2/- | 294.5 ± 15.05 | -21.5 ± 9.19 | 0.201 ± 0.024 |
| **NE/siRNA-961** | +0.1/- | 526.52 ± 82.99 | -43.69 ± 9.30 | 0.67 ± 0.07 |
| | +0.5/- | 350.46 ± 80.41 | -34.98 ± 17.61 | 0.54 ± 0.14 |
| | +2/- | 272.1 ± 6.724 | -32.5 ± 0.96 | 0.236 ± 0.051 |
| **NE/siRNA-980** | +0.1/- | 414.8 ± 106.8 | -41.7 ± 3.38 | 0.646 ± 0.093 |
| | +0.5/- | 307.4 ± 66.62 | -26.7 ± 3.98 | 0.419 ± 0.175 |
| | +2/- | 249.9 ± 16.33 | -25.9 ± 8.44 | 0.164 ± 0.036 |

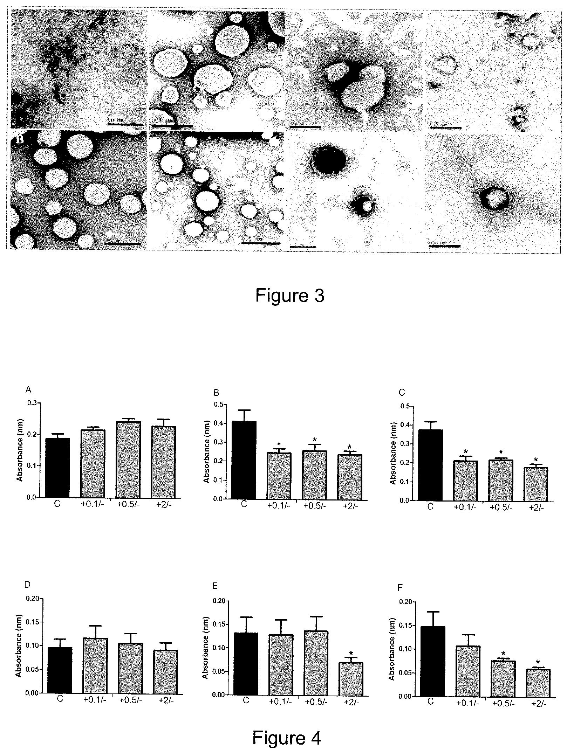

For adsorption studies, NE/siRNA-CD73 complexes were prepared in two different charge ratios (+0.1/-; +0.5/-) and transmission electron microscopy was performed (Figure 3). The analysis revealed that the oil droplets with typical appearance of oil-water emulsion exhibit drops of size around 250-500 nm, in according to the physico-chemical characterization. Interestingly, the NE/siRNA-CD73 complexes showed high electronic density at the interface (Figure 3, frames C-H). It is well established that oligonucleotides interact with uranyl acetate, resulting in regions of dark shading. These data indicate that the siRNA-CD73 sequences were adsorbed at the NE interface.

### Cell transfection procedures:

Transfection is the process of intentional introduction of nucleic acid into cells. Transfection of the C6 glioma cells with siRNA sequences (siRNA-GFP, siRNA-CD73-961 or siRNA-CD73-980) was performed using Lipofectamine® (Lipofectamine RNAiMax, Invitrogen), a transfection agent used to specifically increase the efficiency of RNA lipofection, in C6 glioma cultures seeded in 24-well plates and with approximately 70% confluence according to the manufacturer's instructions.

For the transfection of C6 cells with the NE/siRNA complexes, the complexes were prepared as described above and the C6 glioma cells were transfected using the forward and reverse protocol. For the forward protocol, C6 cells (2x10⁴/well) were seeded in 24-well plates and the transfection mixture was prepared and added to the cells 24 h after the cell seed. For the reverse protocol, the complexes were prepared inside the well of the 24-well culture plate and, afterwards, C6 cells (2x10⁴/well) in culture medium were added. C6 cells were exposed to the complexes for 24, 48 or 72 h.

All results were expressed as mean and standard deviation and statistical analyzes were performed using Analysis of Variance (ANOVA) followed by Tukey's *post-hoc* test (Prism GraphPad Software, USA), considering *p* <0.05 as statistically significant.

### EXAMPLES

### Example 1 - The selective ecto-5'-NT/CD73 inhibitor, AMPCP, decreases cell proliferation of C6 glioma:

The activity of ecto-5'-nucleotidase/CD73 was analyzed in C6 glioma cells compared to astrocytes, in a model of untransformed cell.

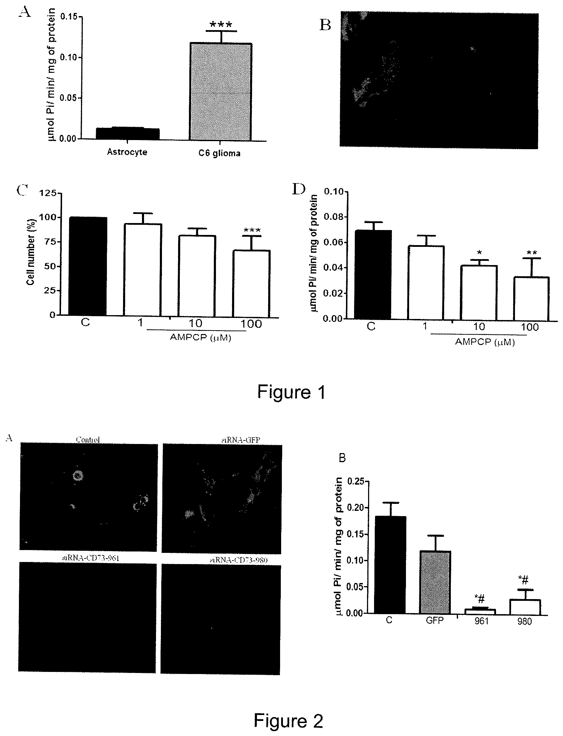

In opposite to astrocytes, C6 glioma showed a high AMPase activity (0.013 ± 0.001 *versus* 0.12 ± 0.01 µmol Pi/min/mg of protein, for astrocytes and C6 cells, respectively; Figure 1, panel A), which was followed by high ecto-5'-nucleotidase/CD73 expression on the surface of tumor cells (Figure 1, panel B).

Therefore, it was evaluated whether the selective ecto-5'nucleotidase/CD73 inhibitor, AMPCP, affects the proliferation of C6 glioma cells. The cells were exposed to increasing concentrations of AMPCP (1, 10 and 100 µM) and after 48 h the cell proliferation was determined by cell counting in a neubauer chamber. In parallel, the activity of the enzyme ecto-5'-nucleotidase/CD73 was analyzed by measuring the hydrolysis of AMP to adenosine (ADO) using the malachite green method.

The treatment of C6 glioma with AMPCP (100 µM) resulted in a 30% decrease in cell proliferation when compared to control (Figure 1, panel C). In addition, the treatment with 10 µM and 100 µM AMPCP reduced the hydrolysis of AMP by 40 and 50%, respectively (Figure 1, panel D).

These data indicate that CD73 is overexpressed in gliomas and that its pharmacological inhibition is important to reduce the proliferation of tumor cells.

### Example 2 - New siRNA-CD73 sequences are efficient to knockdown the ecto-5'-nucleotidase/CD73 expression in glioma cells:

Considering that the ecto-5'-nucleotidase/CD73 overexpression is a positive factor for tumor development, including glioblastoma multiforme (Figure 1), a second finding was that the silencing of this enzyme using interference RNA technology could be an interesting strategy to control glioma progression.

For this purpose, specific siRNA-CD73 sequences were developed (siRNA-CD73-961 and siRNA-CD73-980; Table 1) and their functionality/specificity was assessed by transfecting C6 cells using Lipofectamine® according to the manufacturer's instructions. Following 48 h of cell transfection, CD73 expression and enzyme activity were evaluated by immunocytochemistry and AMP hydrolysis, respectively.

Cells transfected with siRNA-GFP sequences were used as controls. As shown in Figure 2, siRNA-CD73 sequences were efficient in reducing CD73 expression (Figure 2, panel A) and AMP hydrolysis (Figure 2, panel B) when compared to control and cells transfected with siRNA-GFP sequences. Furthermore, the transfection process *per se* did not induce cytotoxicity in the cells.

These results indicate that the siRNA-CD73 sequences designed were efficient and specific to reduce the expression and activity of CD73 in C6 glioma and these sequences were further used for the development of nanoemulsions (NE).

### Example 3 - NE / SiRNA complexes are not cytotoxic for astrocyte culture primary:

Considering that cationic NE has been associated with biological toxicity, the C6 glioma transfection protocol was optimized aiming equalizes high transfection rate and low toxicity.

For this purpose, NE/siRNA-GFP complexes were prepared in three different charge ratios (+0.1/-; +0.5/-; +2/-) and C6 cells were transfected using the forward and reverse protocols, as described in materials and methods. After 24, 48 and 72 h of exposure, cell viability was determined by MTT (Figure 4, panels A-F).

Forward transfection resulted in 50% toxicity in C6 glioma after 48 and 72 h of exposure to the complexes when compared to untreated cells (Figure 4, panels A-C). For reverse transfection, exposure of C6 cells to the NE/siRNA-GFP complexes at +0.1/- and +0.5/- charge ratios for 48 h did not alter cell viability (Figure 4, panel E), while exposure for 72 h resulted in 50% toxicity for +0.5/- and +2/- charge ratios (Figure 4, panel F). Taken together, these data indicate that the reverse transfection protocol resulted in reduced toxicity when compared to the forward protocol. Therefore, the NE/siRNA-GFP complexes in two charge ratios (+0.1/-; +0.5/-) and the reverse protocol for 48 h of exposure was used in the further experiments.

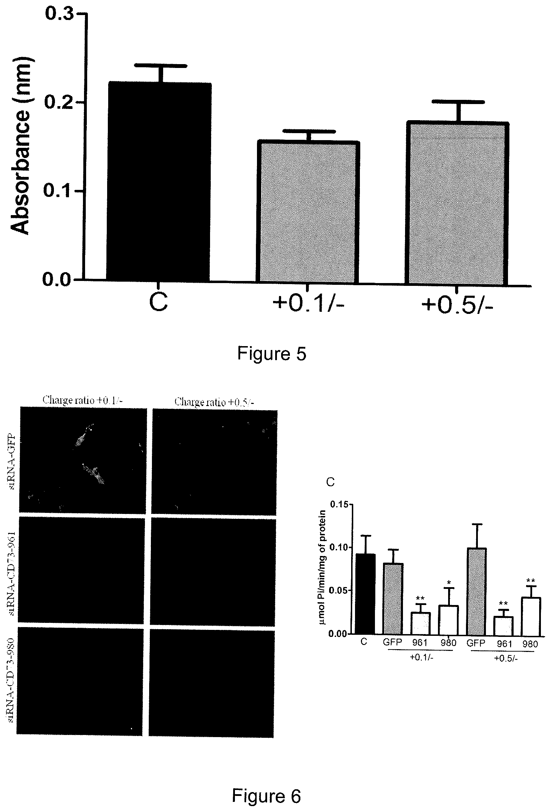

Finally, the toxicity of the NE/siRNA complexes was also evaluated in astrocytes, a model of untransformed glial cell. Astrocytes were exposed to the NE/siRNA-GFP complexes (+0.1/-; +0.5/-) for 48 h and cell viability was determined by MTT. Notably, the complexes did not promote toxicity to astrocytes when compared to the control, indicating the safety of the formulations (Figure 5).

### Example 4 - The NE/siRNA-CD73 complexes are efficient to knockdown ecto-5'-nucleotidase/CD73 expression and activity in C6 glioma:

After the physical-chemical characterization and the optimization of the transfection protocol, the effectiveness of the NE/siRNA-CD73 complexes to knockdown CD73 in C6 glioma was analyzed by immunocytochemistry for CD73 and AMP hydrolysis experiments.

CD73 expression was significantly decreased in cells exposed to NE/siRNA-CD73 complexes when compared to transfected cells with scramble sequences (NE/siRNA-GFP) (Figures 6, panels A and B). Similarly, AMPase activity was reduced in silenced cells (reduction of 70% and 63% for siRNA-CD73-961 and siRNA-CD73-980, respectively; Figure 6, panel C). Therefore, the NE/siRNA-CD73-961 and NE/siRNA-CD73-980 complexes were efficient and selective to knockdown CD73 expression in C6 glioma.

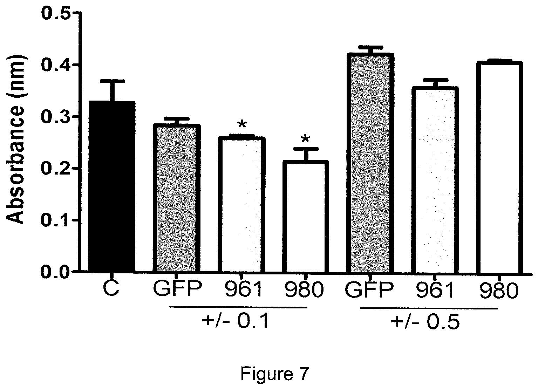

Furthermore, the transfection of C6 cells with NE/siRNA-CD73-961 and 980 complexes using +0.1/- charge ratio resulted in 20 and 35% reduction of glioma cell viability, respectively (Figure 7). Taken together, these data suggest that cationic NEs were efficient to deliver siRNA-CD73 sequences to C6 glioma cells. In addition, CD73 is an interesting target for the treatment of gliomas.

The depositor, when filing this patent application before the competent body/guarantor, seeks and intends to: (i) appoint the inventors with respect to their respective moral rights; (ii) indicate unequivocally that it is the holder of industrial secrecy and holder of any form of intellectual property that may derive from it and the depositor wishes; (iii) describe in detail the content subject to the secret, proving its existence on the physical and legal levels; (iv) establish the relationship between the examples/achievements and the inventive concept according to the depositor's cognition and context, to clearly demonstrate the scope of his tutored and/or tutelable intangible asset; (v) request and obtain the additional rights foreseen for patents, if the depositor chooses to proceed with the administrative procedure until the end.

Right away, it is warned that any commercial use requires authorization from the owner/holder and that the unauthorized use entails sanctions provided for by law. In this context, given the broad detail according to which the concept and examples were revealed by the depositor, those skilled in the art will be able, without much effort, to consider other ways of carrying out the present invention in ways not identical to those merely exemplified above. However, such forms are or may be considered to fall within the scope of one or more of the attached claims.

## Claims

1. Nanometric pharmaceutical composition **characterized by** comprising liposomes or nanoemulsion containing interfering RNA filaments.

2. Nanometric pharmaceutical composition according to claim 1, **characterized by** the interfering RNA strands are composed of two identical sequences of 19 nucleotides in inverted orientation, separated by a space of 9 base pairs, defined according to SEQ ID No: 1, SEQ ID No: 2 and SEQ ID No: 3.

3. Nanometric pharmaceutical composition according to any one of claims 1 to 2, **characterized by** comprising additionally a pharmaceutically acceptable excipient.

4. Nanometric pharmaceutical composition according to any one of claims 1 to 3, **characterized by** being in the form of an intratumoral, parenteral injection or nasal spray.

5. Use of the nanometric pharmaceutical composition as defined in any one of claims 1 to 4, **characterized** to be used to prepare a drug to silence genes responsible for the expression of overexposed proteins in tumors.

6. Use of the nanometric pharmaceutical composition according to claim 5, **characterized by** the fact that the silenced gene is 5'-nucleotidase-ecto.

7. Use of the nanometric pharmaceutical composition according to claim 5, **characterized by** the protein to be regulated is ecto-5'-nucleotidase/CD73.

8. Use of the nanometric pharmaceutical composition according to claim 5, **characterized in that** the tumor is glioblastoma multiforme.

9. Use of the nanometric pharmaceutical composition according to any one of claims 5 to 8, **characterized by** it is administered in the form of an intratumoral, parenteral injection or nasal spray.
